# EUROPÄISCHE PATENTSCHRIFT

(11) **EP 1 485 338 B1**
(45) Veröffentlichungstag und Bekanntmachung des Hinweises auf die Patenterteilung: **12.11.2008**
(21) Anmeldenummer: 03743820.7
(22) Anmeldetag: 27.02.2003
(51) Int. Cl.: C07C 43/21, C07C 49/755

(54) **ALKOXY-SUBSTITUIERTE INDANONE UND DEREN HERSTELLUNG**
ALKOXY-SUBSTITUTED INDANONES AND THE PRODUCTION THEREOF
INDANONES SUBSTITUES PAR ALCOXY ET LEUR PRODUCTION

(30) Priorität: 11.03.2002 DE 10210623
(43) Veröffentlichungstag der Anmeldung: 15.12.2004
(73) Patentinhaber: Symrise GmbH & Co. KG, 37603 Holzminden (DE)
(72) Erfinder: KOCH, Oskar, 37079 Göttingen (DE)
(74) Vertreter: Eisenführ, Speiser & Partner
(86) Internationale Anmeldenummer: PCT/EP2003/001987
(87) Internationale Veröffentlichungsnummer: WO 2003/076379

(56) Entgegenhaltungen:
- EP-A- 1 000 950
- WO-A-02/38537
- DATABASE CROSSFIRE BEILSTEIN [Online] Beilstein Institut zur Förderung der Chemischen Wissenschaften, Frankfurt am Main, DE; retrieved from XFIRE Database accession no. 5505898 XP002242444 & BULL. SOC. CHIM. BELG., Bd. 90, Nr. 8, 1981, Seiten 847-848,
- DATABASE CROSSFIRE BEILSTEIN [Online] Beilstein Institut zur Förderung der Chemischen Wissenschaften, Frankfurt am Main, DE; retrieved from XFIRE Database accession no. 8762625 XP002242445 & BULL. CHEM. SOC. JP., Bd. 73, Nr. 12, 2000, Seiten 2779-2782,
- DATABASE CROSSFIRE BEILSTEIN [Online] Beilstein Institut zur Förderung der Chemischen Wissenschaften, Frankfurt am Main, DE; retrieved from XFIRE Database accession no. 2209219 XP002242446 & J. AMER. CHEM. SOC., Bd. 88, 1966, Seiten 5809-5816,
- DATABASE CROSSFIRE BEILSTEIN [Online] Beilstein Institut zur Förderung der Chemischen Wissenschaften, Frankfurt am Main, DE; retrieved from XFIRE Database accession no. 2441487 XP002242447 & J. AMER. CHEM. SOC., Bd. 102, Nr. 17, 1980, Seiten 5618-5626,
- DATABASE CROSSFIRE BEILSTEIN [Online] Beilstein Institut zur Förderung der Chemischen Wissenschaften, Frankfurt am Main, DE; retrieved from XFIRE Database accession no. 3272305 XP002242457 & HELV. CHIM. ACTA, Bd. 42, 1959, Seiten 2111-2117,
- EISENBRAUN ET AL.: "Polyalkyl Aromatic Hydrocarbons. II. Cyclialkylation of Benzoid Hydrocarbons with Isoprene" J. ORG. CHEM., Bd. 33, 1968, Seiten 2000-2008, XP002242443
- DATABASE CROSSFIRE BEILSTEIN [Online] Beilstein Institut zur Förderung der Chemischen Wissenschaften, Frankfurt am Main, DE; retrieved from XFIRE Database accession no. 2209496 XP002242448 & BULL. SOC. CHIM. FR., 1947, Seiten 812-815,
- DATABASE CROSSFIRE BEILSTEIN [Online] Beilstein Institut zur Förderung der Chemischen Wissenschaften, Frankfurt am Main, DE; retrieved from XFIRE Database accession no. 3266549 XP002242451 & J. CHEM. SOC., 1951, Seiten 83-86,
- DATABASE CROSSFIRE BEILSTEIN [Online] Beilstein Institut zur Förderung der Chemischen Wissenschaften, Frankfurt am Main, DE; retrieved from XFIRE Database accession no. 2372513 XP002242449 & TETRAHEDRON, Bd. 30, 1974, Seiten 2887-2890,
- DATABASE CROSSFIRE BEILSTEIN [Online] Beilstein Institut zur Förderung der Chemischen Wissenschaften, Frankfurt am Main, DE; retrieved from XFIRE Database accession no. 2444004 XP002242450 & YAKUGAKU ZASSHI, Bd. 76, 1956, Seiten 163-166,
- DATABASE CROSSFIRE BEILSTEIN [Online] Beilstein Institut zur Förderung der Chemischen Wissenschaften, Frankfurt am Main, DE; retrieved from XFIRE Database accession no. 6204342 XP002242452 & Z. NATURFORSCH. B. ANORG. CHEM. ORG. CHEM., Bd. 39, Nr. 12, 1984, Seiten 1801-1805,

## Beschreibung

Die vorliegende Erfindung betrifft die Herstellung und Verwendung Alkoxy-substituierter Indanone gemäß der Ansprüche 1 bis 5.

1-Indanone sind wichtige Zwischenprodukte zur Herstellung von pharmazeutischen Produkten (EP-A 421 759 und EP-A 404 536) sowie von UV-Filtern (EP-A 823 418, DE-A 10055940.9-44).

Die Herstellung von 1-Indanonen kann durch Oxidation von entsprechend substituierten Indanen mit Oxidationsmitteln wie z.B. Sauerstoff oder Luft in Anwesenheit von Metallkatalysatoren wie z.B. Co-Salzen erfolgen (J. prakt. Chem. 334, 373 (1992)).

Diese Oxidation wird z.B. in EP-A 162 465 anhand von Tri-und Tetramethyl-indanen mit Hilfe von Chrom- und Kobaltacetoacetat beschrieben.

Desweiteren sind derartige Oxidationen auch mit Imiden wie z.B. N-Hydroxy-phthalimid möglich (J. Org. Chem. 60, 3934 (1995)).

In der Literatur sind für die Herstellung der Indane die Umsetzung von alkyl-substituierten Aromaten wie z.B. Toluol oder Xylol (J. Org. Chem. 54, 1418 (1989)) mit Isopren beschrieben. Phenole oder Kresole können analog, wie dies in DE-A 2 603 835 beschrieben wird, umgesetzt werden, wobei die Ausbeuten allerdings niedrig sind.

Alkoxy-substituierte Indane sind in EP-A 286 523 und EP-A 807 850 in einer breiten allgemeinen Formel umfasst, es werden dort jedoch weder explizit Alkoxy-substituierte Indane noch deren Herstellung beschrieben.

Es bestand somit die Aufgabe, ein geeignetes Herstellungsverfahren für Alkoxy-substituierte 1-Indanone zu finden.

Überraschenderweise wurde gefunden, dass Alkoxy-substituierte Aromaten bei der Umsetzung mit Isopren (2-Methyl-1,3-butadien) deutlich bessere Ausbeuten liefern als dies bei Phenolen oder Kresolen der Fall ist.

Offenbart werden Alkoxy-substituierte Indane der Formel (**II**) 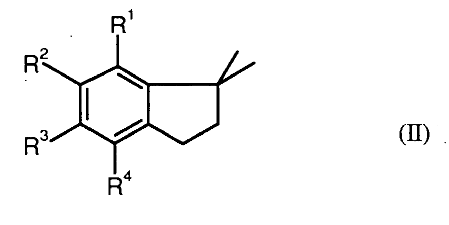 wobei
- R¹, R², R³ und R⁴: -unabhängig voneinander- Wasserstoff, C₁-C₈-Alkyl oder C₁-C₈-Alkoxy sein können, mit der Maßgabe, dass mindestens einer dieser Reste C₁-C₈-Alkoxy ist.

Bevorzugte offenbarte Alkoxy-substituierte Indane sind: 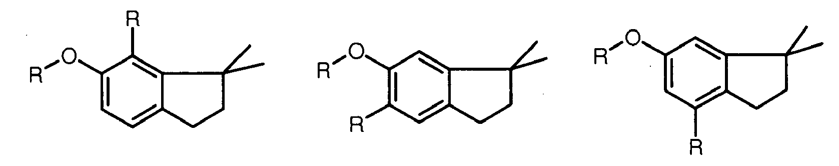 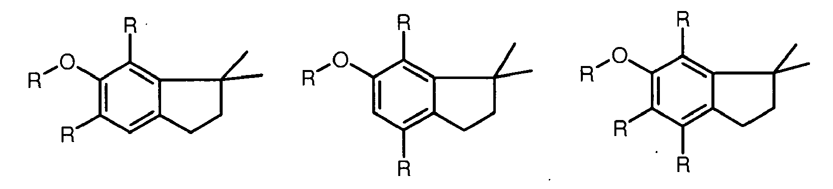 wobei die Substituenten R -unabhängig voneinander- Methyl, Ethyl, Propyl, i-Propyl, n-Butyl, i-Butyl, tert.-Butyl, n-Pentyl oder i-Pentyl bedeuten können.

Besonders bevorzugte Verbindungen sind:

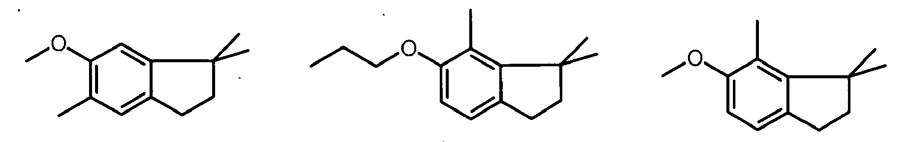

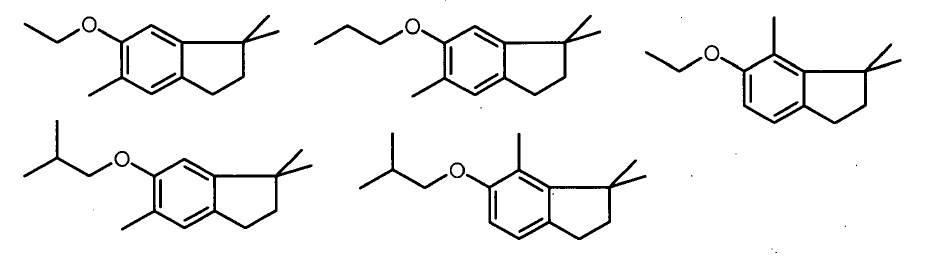

Ganz besonders bevorzugte Verbindungen sind:

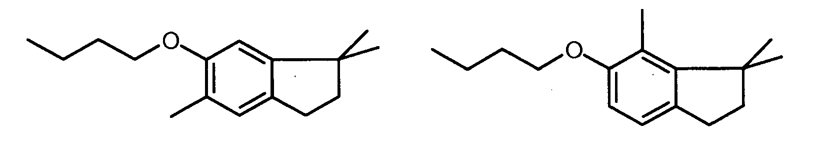

Weiterhin offenbart wird die Herstellung von Verbindungen der Formel (**II**) aus Alkoxy-substituierten Aromaten der Formel (**I**) und Isopren in Gegenwart eines sauren Katalysators sowie die Verwendung der Verbindungen der Formel (II) zur Herstellung der entsprechenden Alkoxy-substituierten 1-Indanone (**III**).

Folgendes Reaktionsschema kann das offenbarte Verfahren zur Herstellung der Verbindungen der Formel (II) verdeutlichen: 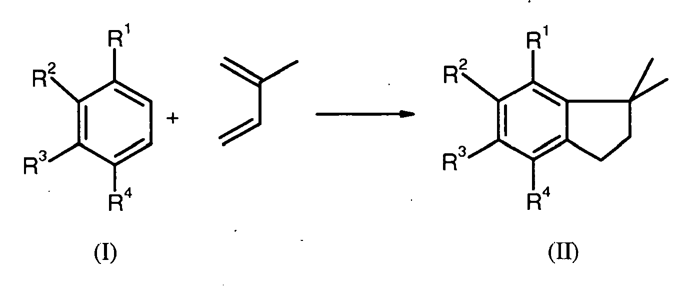 wobei
- R¹, R², R³ und R⁴: -unabhängig voneinander- Wasserstoff, C₁-C₈-Alkyl oder C₁-C₈-Alkoxy sein können, mit der Maßgabe, dass mindestens einer dieser Reste C₁-C₈-Alkoxy ist.

Bevorzugt einzusetzende Alkoxy-substituierten Aromaten sind: 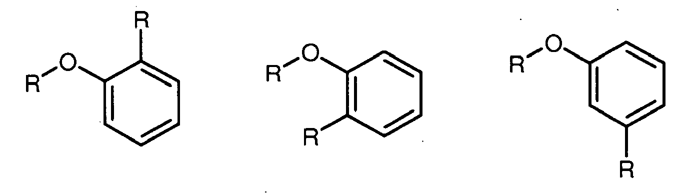 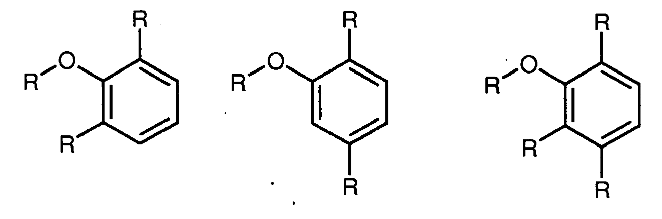 wobei die Substituenten R -unabhängig voneinander- Methyl, Ethyl, Propyl, i-Propyl, n-Butyl, i-Butyl, tert.-Butyl, n-Pentyl oder i-Pentyl bedeuten können.

Besonders bevorzugte Alkoxy-substituierten Aromaten sind:

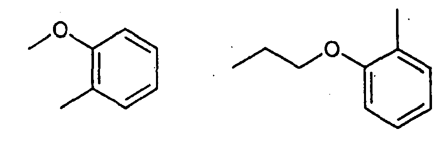

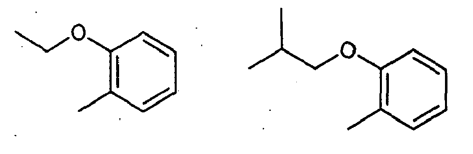

Ganz besonders bevorzugt ist:

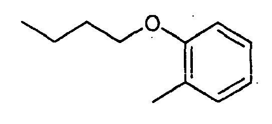

Die Umsetzung der Alkoxy-substituierten Aromaten (I) mit Isopren erfolgt in Gegenwart saurer Katalysatoren. Es können generell übliche Friedel-Crafts-Katalysatoren eingesetzt werden. Als saure Katalysatoren können z.B. anorganische Säuren wie Phosphorsäure, Schwefelsäure oder organische Säuren wie Methansulfonsäure eingesetzt werden. Weitere geeignete Katalysatoren sind Lewis-Säuren wie beispielsweise AlCl₃, ZnCl₂, FeCl₃, TiCl₄ und BF₃-Addukte. Bevorzugter Katalysator ist Schwefelsäure, besonders bevorzugt ist 70 - 95 gew.%ige Schwefelsäure.

Das molare Verhältnis zwischen den Alkoxy-substituierten Aromaten (I) und Isopren liegt bevorzugt zwischen 7:1 und 1:2, besonders bevorzugt zwischen 3:1 und 1:1.

Das gewichtsbezogene Verhältnis zwischen den Alkoxy-substituierten Aromaten (I) und Schwefelsäure liegt bevorzugt zwischen 5:1 und 1:1, besonders bevorzugt zwischen 3:1 und 2:1.

Die Reaktionstemperatur liegt vorteilhafterweise zwischen 0 und 100°C, bevorzugt zwischen 20 und 80°C und insbesonders bevorzugt zwischen 30 und 50°C. Die Reaktionszeit liegt vorteilhafterweise zwischen 10 und 300 min, bevorzugt zwischen 30 und 120 min und insbesonders bevorzugt zwischen 40 und 90 min.

Ein Gegenstand der vorliegenden Erfindung ist die Herstellung von Alkoxy-substituierten 1-Indanonen durch Oxidation von Verbindungen der Formel (II) gemäß Anspruch 1, neue Alkoxy-substituierte 1-Indanone gemäß Anspruch 2 und die Verwendung neuer Alkoxy-substituierter 1-Indanone zur Herstellung von pharmazeutischen oder agrochemischen Wirkstoffen gemäß Anspruch 4 sowie zur Herstellung von UV-Filtern gemäß Anspruch 5.

Folgendes Schema kann das Verfahren zur Herstellung der Alkoxy-substituierten 1-Indanone der Formel (III) verdeutlichen: 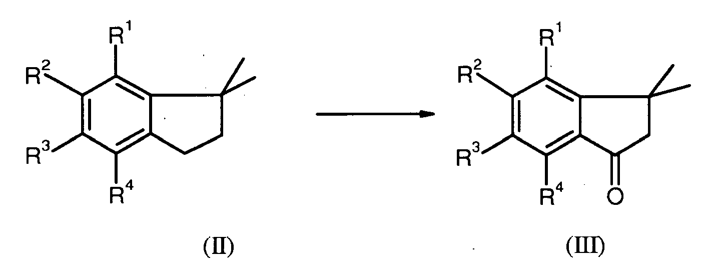 wobei
- R¹ bis R⁴: die gemäß Anspruch 1 genannte Bedeutung haben.

Generell kann diese Oxidation in Benzylstellung nach literaturbekannten Verfahren erfolgen.

Bevorzugt wird die Oxidation mit Sauerstoff durchgeführt, wobei der Sauerstoff auch mit anderen Gasen verdünnt sein kann. Vorteilhaft ist die Verdünnung mit Inertgasen, besonders vorteilhaft ist die Oxidation mit Luft.

Die Umsetzung kann unter erhöhtem Druck erfolgen. Typischerweise erfolgt die Reaktion bei Drücken im Bereich von 1 bis 50 bar abs.

Diese Oxidation kann insbesondere in Gegenwart von Verbindungen der Metalle Mangan, Eisen, Kobalt, Chrom, Nickel oder Kupfer durchgeführt werden. Bevorzugt sind Halogenide, Nitrate, Sulfate, Oxide, Acetate oder Wolframate der genannten Metalle. Weiterhin können die genannten Metalle als Komplexe mit Chelatbildnern wie z.B. Acetylacetonate, Phthalocyaninen, Porphyrinen, Azaporphyrinen eingesetzt werden. Die Metallverbindungen können als solche oder auch auf Trägem verwendet werden. Weiterhin können die genannten Katalysatoren einzeln oder in Mischungen eingesetzt werden.

Bevorzugte Metalle sind Nickel, Kobalt und Kupfer. Bevorzugte Metallverbindungen sind die Halogenide, Sulfate, Acetate und Acetylacetonate. Besonders bevorzugte Metallverbindungen sind Co-(II)-acetat, Co-(II)-acetylacetonat, Co-(III)-acetyl-acetonat, Ni-(II)-acetat und Ni-(II)-acetylacetonat.

Die Einsatzmenge der Metallverbindung kann in weiten Bereichen variiert werden und beträgt üblicherweise zwischen 0,00001 und 15 mol%, bevorzugt 0,25 bis 10 mol%.

Diese Oxidation kann ebenfalls in Gegenwart von N-Hydroxy-imiden durchgeführt werden. Bevorzugte N-Hydroxy-imide sind N-Hydroxy-succinimid, N-Hydroxymaleinimid und N-Hydroxy-phthalimid, besonders bevorzugt ist N-Hydroxy-phthalimid. Die Einsatzmenge der N-Hydroxy-imide kann in weiten Bereichen variiert werden und beträgt üblicherweise zwischen 0,001 und 15 mol%, bevorzugt 1 bis 10 mol%.

Ebenfalls vorteilhaft ist die Durchführung der Oxidation in Gegenwart einer Verbindung der oben genannten Metalle und einem N-Hydroxy-imid, bevorzugt einer Ni-(II)-, Co-(II)- oder Co(III)-Verbindung und N-Hydroxy-phthalimid, wobei Co-(II)-acetat, Co-(II)-acetylacetonat, Co-(III)-acetylacetonat, Ni-(II)-acetat und Ni-(II)-acetylacetonat bevorzugt sind.

Die Reaktionstemperaturen liegen typischerweise bei 0 bis 200°C, bevorzugt bei 20 bis 120°C, besonders bevorzugt bei 30 bis 90°C.

Je nach Konsistenz von Edukten oder Produkten kann ein organisches Verdünnungsmittel verwendet werden. Als Verdünnungsmittel können beispielsweise Kohlenwasserstoffe, Ether, Alkohole, Alkyl- oder Arylnitrile sowie organische Säuren verwendet werden. Besonders geeignet sind niedere Alkohole wie beispielsweise Methanol, Ethanol oder i-Propanol, niedere organische Säuren wie beispielsweise Essigsäure sowie Alkyl- oder Arylnitrile wie beispielsweise Acetonitril oder Benzonitril.

Weiterhin kann die Oxidation unter den Bedingungen der Phasentransfer-Katalyse durchgerührt werden, indem man zu den nicht mit Wasser mischbaren Alkoxy-substituierten Indanen die oben genannten Metallsalze als wässrige Lösung unter Zugabe eines Phasentransfer-Katalysators zusetzt. Dabei können die Alkoxy-substituierten Indane allein oder in einem Lösungsmittel zum Einsatz kommen.

Vorteilhaft lässt sich die Reaktion z.B. in Benzonitril als organische Phase sowie einer Lösung von Co-(II)-chlorid, Cu-(II)-nitrat und Tetrabutylammoniumbromid als wässrige Phase durchführen.

Offenbart werden neue Alkoxy-substituierte 1-Indanone der Formel (IV) sowie deren Herstellung nach dem oben beschriebenen Verfahren. 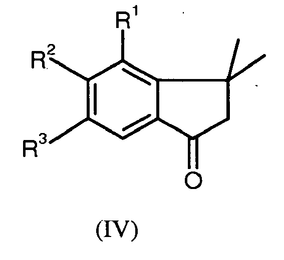 wobei
- R¹, R² und R³: die oben genannte Bedeutung halben.

Bevorzugte Alkoxy-substituierte 1-Indanone sind:

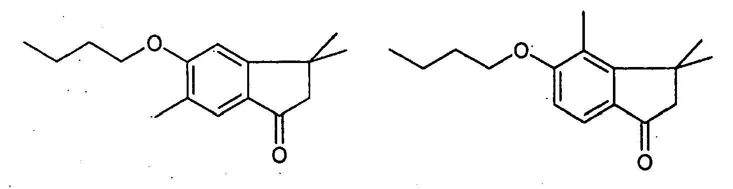

Folgende Beispiele können die Erfindung erläutern:

### Beispiele

### Beispiel 1:

### 3,3,6-Trimethyl-5-methoxy-indan (nicht erfindungsgemäß) bzw. 3,3,4-Trimethyl-5-methoxy-indan (nicht erfindungsgemäß)

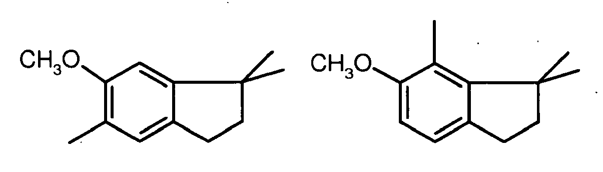

300 g Schwefelsäure (85 gew.-%ig) werden unter Rühren vorgelegt und innerhalb von 50 min mit einem Gemisch aus 732 g o-Kresyl-methylether und 153 g Isopren versetzt. Dabei hält man die Temperatur durch Kühlen auf max. 30°C. Man rührt 20 min bei dieser Temperatur nach, gibt 400 g Wasser hinzu, trennt die Phasen und wäscht mit Natriumbicarbonat neutral. Nach Abdestillieren des überschüssigen o-Kresyl-methyl-ethers erhält man 300 g des Isomerengemischs (Verhältnis 3: 1), was einer Ausbeute von 70 % der Theorie entspricht.

Die beiden Isomere werden an einer Im-Füllkörperkolonne destillativ getrennt und können einzeln zur Oxidation eingesetzt werden.

### Beispiel 2:

### 3,3,6-Trimethyl-5-butoxy-indan (nicht erfindungsgemäß) bzw. 3,3,4-Trimethyl-5-butoxy-indan (nicht erfindungsgemäß)

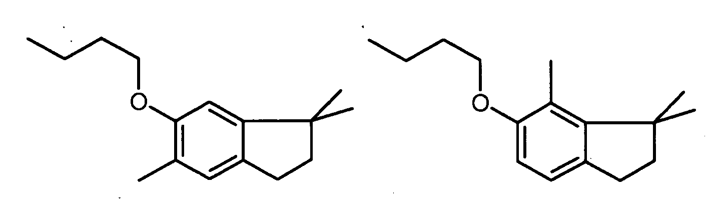

Die Umsetzung erfolgt in Analogie zu Beispiel 1, wobei o-Kresyl-butylether eingesetzt wird. Die Ausbeute liegt bei 70 % der Theorie.

### Herstellung von 2-Methyl-butoxy-benzol (o-Kresyl-butylether)

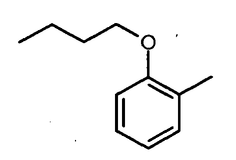

540 g (5,0 mol) o-Kresol, 500 g n-Butanol sowie 560 g (5,0 mol) 50 gew.-%ige Kalilauge werden vorgelegt und am Rückfluss erhitzt, wobei das Wasser azeotrop entfernt wird. Anschließend dosiert man bei Rückfluss innerhalb von 2h 463 g (5,0 mol) n-Butylchlorid zu, wobei weiter Wasser ausgekreist wird. Man rührt nach Dosierung noch weitere 2h, kühlt auf 80°C ab, hydrolysiert mit 800 g Wasser und stellt mit 50 gew.-%iger Schwefelsäure auf pH 4 ein. Die Phasen werden bei 70°C getrennt. Nach Destillation erhält man 794 g Produkt mit einer Reinheit von 94 %.
Ausbeute: 90 % der Theorie.

### Beispiel 3:

### 3,3,6-Trimethyl-5-butoxy-indan-1-on

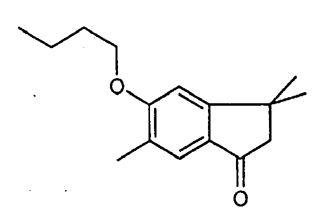

100 g 3,3,6-Trimethyl-5-butoxy-indan werden in 400g Essigsäure gelöst und mit 1 g Co-II-acetat versetzt. Man erhitzt unter Rühren auf 40°C und leitet durch diese Lösung 10 Stunden Sauerstoff. Nach Abkühlen auf Raumtemperatur gibt man 500 g Wasser und 500 g Methyl-tert.-butylether hinzu. Nach Phasentrennung wäscht man die organische Phase nochmals mit 200 g Wasser nach und destilliert an einer 30 cm-Füllkörperkolonne. Man erhält 80 g 3,3,6-Trimethyl-5-butoxy-indan-1-on, was einer Ausbeute von 75 % d.Th. entspricht. Die Verbindung läßt sich aus Heptan umkristallisieren und man erhält einen weißen Festsfoff, Schmelzpunkt: 59°C.

### Beispiel 4:

### 3,3,4-Trimethyl-5-butoxy-indan-1-on

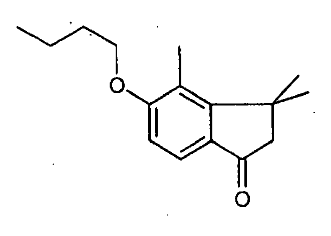

Die Umsetzung erfolgt in Analogie zu Beispiel 3, wobei 3,3,4-Trimethyl-5-butoxy-indan eingesetzt wird. Die Ausbeute liegt bei 70 % der Theorie.

## Patentansprüche

1. Verfahren zur Herstellung eines Alkoxy-substituierten Indanons der Formel (III) durch Oxidation eines Alkoxy-substituierten Indans der Formel (II) 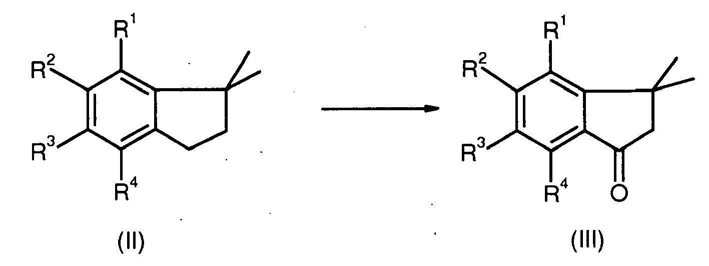 wobei
R¹, R², R³ und R⁴ - unabhängig voneinander - Wasserstoff, C₁-C₈-Alkyl oder C₁-C₈-Alkoxy bedeuten, mit der Maßgabe, dass mindestens einer dieser Reste C₁-C₈-Alkoxy ist,
**dadurch gekennzeichnet, dass**
die Verbindung der Formel (II) ausgewählt wird aus einer der Formeln 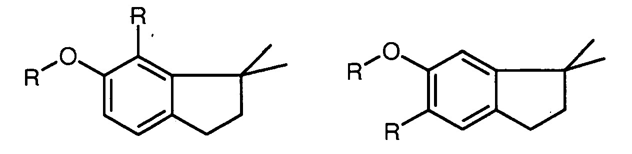 wobei
die Substituenten R -unabhängig voneinander- Methyl, Ethyl, Propyl, i-Propyl, n-Butyl, i-Butyl, tert.-Butyl, n-Pentyl oder i-Pentyl bedeuten,
mit der Maßgabe, dass die folgende Verbindung ausgenommen ist:

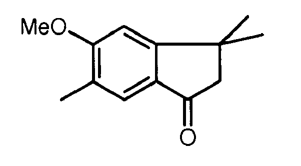

2. Verbindungen der Formel (III) 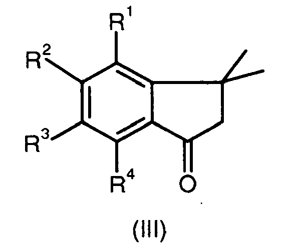 erhältlich durch Oxidation eines Alkoxy-substituierten Indans der Formel (II) 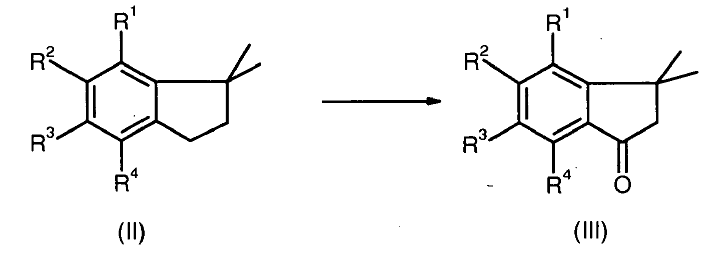 wobei
R¹, R², R³ und R⁴ - unabhängig voneinander - Wasserstoff, C₁-C₈-Alkyl oder C₁-C₈-Alkoxy bedeuten, mit der Maßgabe, dass mindestens einer dieser Reste C₁-C₈-Alkoxy ist,
**dadurch gekennzeichnet, dass**
die Verbindung der Formel (II) ausgewählt wird aus einer der Formeln 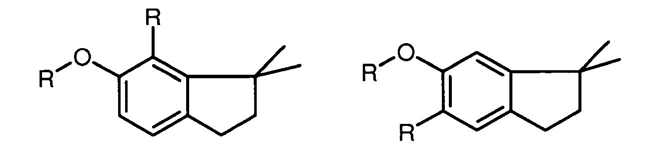 wobei
die Substituenten R -unabhängig voneinander- Methyl, Ethyl, Propyl, i-Propyl, n-Butyl, i-Butyl, tert.-Butyl, n-Pentyl oder i-Pentyl bedeuten,
mit der Maßgabe, dass die folgende Verbindung ausgenommen ist:

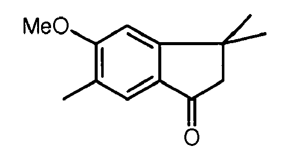

3. Verbindungen nach Anspruch 2, **dadurch gekennzeichnet, dass** es sich dabei um die Verbindungen der folgenden Formeln handelt:

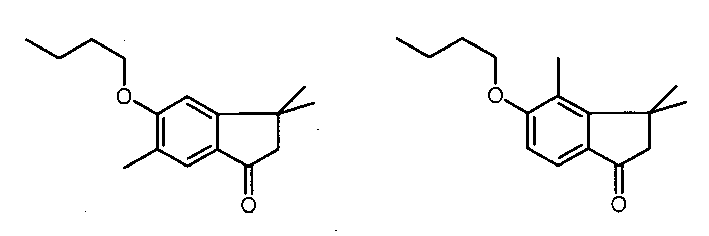

4. Verwendung von Verbindungen nach Anspruch 2 oder 3 zur Herstellung von pharmazeutischen oder agrochemischen Wirkstoffen.

5. Verwendung von Verbindungen nach Anspruch 2 oder 3 zur Herstellung von UV-Filtern.

## Claims

1. Process for the production of an alkoxy-substituierted indanone of the formula (III) by oxidation of an alkoxy-substituierted indane of the formula (II) 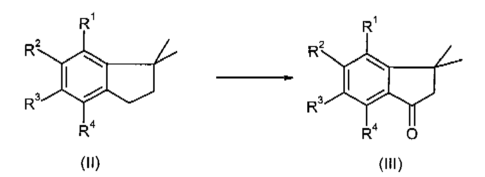 wherein
R¹, R², R³ and R⁴ - independently from each other - represent hydrogen, C₁-C₈-alkyl or C₁-C₈-alkoxy, with the proviso, that at least on of the substituents is C₁-C₈-alkoxy,
**characterized in that**
the compound of the formula (II) is selected from one of the formulas 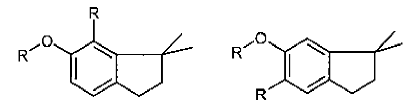 wherein
the substituents R - independently from each other - represent methyl, ethyl, propyl, i-propyl, n-butyl, i-butyl, tert.-butyl, n-pentyl oder i-pentyl,
with the proviso that the following compound is disclaimed:

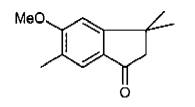

2. Compounds of the formula (III) 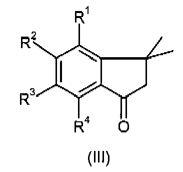 obtainable by oxidation of an alkoxy-substituierted indane of the formula (II) 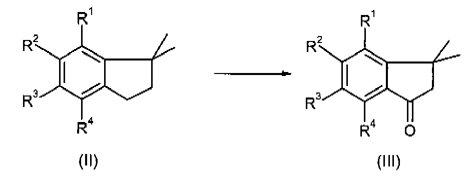 wherein
R¹, R², R³ and R⁴ - independently from each other - represent hydrogen, C₁-C₈-alkyl or C₁-C₈-alkoxy, with the proviso that at least one of the substituents is C₁-C₈-alkoxy,
**characterized in that**
the compound of the formula (II) is selected from one of the formulas 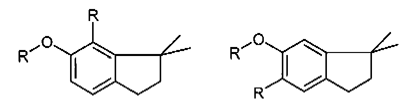 wherein
the substituents R - independently from each other - represent methyl, ethyl, propyl, i-propyl, n-butyl, i-butyl, tert.-butyl, n-pentyl oder i-pentyl,
with the proviso that the following compund is disclaimed:

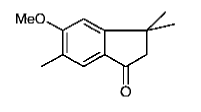

3. Compounds according to claim 2, **characterized in that** the compounds are of the following formulas:

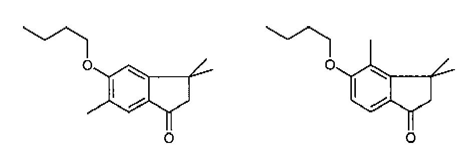

4. Use of compounds according to claim 2 or 3 in the production of pharmaceutical or agrochemical active agents.

5. Use of compounds according to claim 2 or 3 in the production of UV-filters.

## Revendications

1. Procédé pour produire une indanone alcoxy-substituée de formule (III) par oxydation d'un indane alcoxy-substitué de formule (II) 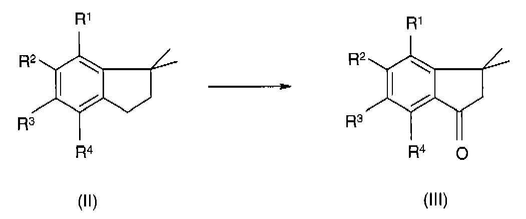 où
R¹, R², R³ et R⁴ représentent indépendamment les uns des autres l'hydrogène, C₁-C₈-alkyle ou C₁-C₈-alcoxy, avec la condition qu'au moins l'un de ces groupements soit C₁-C₈-alcoxy,
**caractérisé en ce que** le composé de formule (II) est choisi parmi l'une des formules 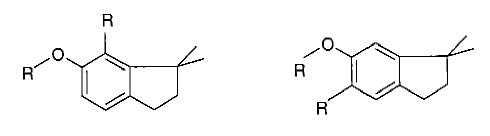 où
les substituants R représentent indépendamment l'un de l'autre méthyle, éthyle, propyle, i-propyle, n-butyle, i-butyle, tert-butyle, n-pentyle ou i-pentyle,
avec la condition que le composé suivant soit exclu :

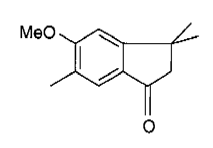

2. Composés de formule (III) 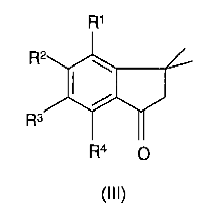 pouvant être obtenus par oxydation d'un indane alcoxy-substitué de formule (II) 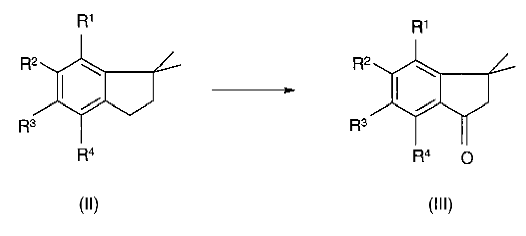 où
R¹, R², R³ et R⁴ représentent indépendamment les uns des autres l'hydrogène, C₁-C₈-alkyle ou C₁-C₈-alcoxy, avec la condition qu'au moins l'un de ces groupements soit C₁-C₈-alcoxy,
**caractérisés en ce que** le composé de formule (II) est choisi parmi l'une des formules 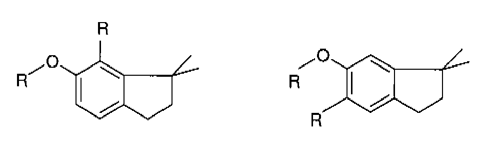 où
les substituants R représentent indépendamment l'un de l'autre méthyle, éthyle, propyle, i-propyle, n-butyle, i-butyle, tert-butyle, n-pentyle ou i-pentyle,
avec la condition que le composé suivant soit exclu :

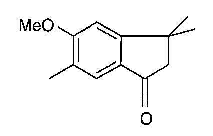

3. Composés selon la revendication 2 **caractérisés en ce qu'**il s'agit des composés des formules suivantes :

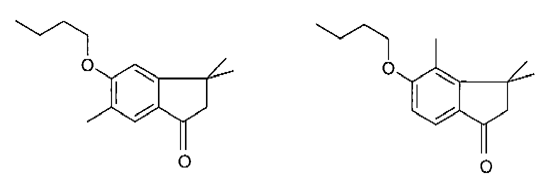

4. Utilisation de composés selon la revendication 2 ou 3 pour la production de principes actifs pharmaceutiques ou agrochimiques.

5. Utilisation de composés selon la revendication 2 ou 3 pour la production de filtres UV.
